# EUROPÄISCHE PATENTSCHRIFT

(11) **EP 0 822 782 B1**
(45) Veröffentlichungstag und Bekanntmachung des Hinweises auf die Patenterteilung: **31.10.2001**
(21) Anmeldenummer: 96911919.7
(22) Anmeldetag: 24.04.1996
(51) Int. Cl.: A61B 17/22

(54) **INTRAKORPORALES BEHANDLUNGSSYSTEM**
INTRACORPORAL TREATMENT SYSTEM
SYSTEME DE TRAITEMENT INTRACORPOREL

(30) Priorität: 24.04.1995 DE 19514440; 26.09.1995 DE 19535828
(43) Veröffentlichungstag der Anmeldung: 11.02.1998
(73) Patentinhaber: Karl Storz GmbH & Co. KG, 78532 Tuttlingen (DE)
(72) Erfinder: IRION, Klaus, D-78576 Liptingen (DE); LEIBERSPERGER, Wolfgang, D-78532 Tuttlingen (DE)
(74) Vertreter: Münich, Wilhelm, Dr.
(86) Internationale Anmeldenummer: DE9600720
(87) Internationale Veröffentlichungsnummer: WO9633661

(56) Entgegenhaltungen:
- US-A- 3 913 584
- US-A- 3 913 585
- US-A- 5 176 688
- US-A- 5 449 363

## Beschreibung

### Technisches Gebiet

Die Erfindung bezieht sich auf ein intrakorporales Behandlungssystem zur Zertrümmerung von Konkrementen, wie Steinen im harnableitenden Trakt, Kalkablagerungen in Gefäßen oder Knochenzement gemäß dem Oberbegriff des Patentanspruchs 1.

### Stand der Technik

Ein intrakorporales Behandlungssystem dieser Art ist aus der US-A-5 160 336 bzw. der weitgehend inhaltsgleichen EP 0 317 507 B1 bekannt.

Weitgehend ähnliche Behandlungssysteme sind aus der DE 38 26 414 A1, der DE 43 13 768 A1, der OE-Patentschrift 309 663 oder der OE-Patentschrift 321 448 bekannt.

Die aus der US-A-5 160 336 oder der EP 0 317 507 B1 bekannten intrakorporalen Behandlungssysteme weisen eine Stoßeinheit auf, die in einer Beschleunigungsstrecke als Stoßelement ein Projektil derart beschleunigt, daß es auf ein Target auftrifft. Die Beschleunigungsstrecke ist dabei ein Rohr, in der das Projektil hin- und her bewegbar ist. Durch einen Lufteinlaß, der an dem dem Target entgegengesetzten Ende des Rohres vorgesehen ist, kann das Projektil mittels Preßluft oder dgl. beschleunigt werden, so daß es mit großer kinetischer Energie auf das Target auftrifft.

Das Target, das bei dem bekannten System ein "stoßübertragendes Fenster" am distalen Ende der Beschleunigungsstrecke ist, überträgt die Bewegungsenergie des Projektils auf den Stoßweiterleiter, der hierdurch abrupt, d.h. stoßförmig verschoben bzw. elongiert wird. Der Stoßweiterleiter kann beispielsweise ein hochfester Metalldraht sein. Durch die Auslenkung des Stoßweiterleiters wird das zu zertrümmernde Konkrement, also beispielsweise ein Harnstein, oder dgl. zerstört.

Bei dem aus der DE 38 26 414 A1 bekannten Behandlungssystem ist ein Ultraschall-Vibrator vorgesehen, der die Energie erzeugt, durch die der Stoßweiterleiter beaufschlagt wird.

In der DE 43 13 768 A1. ist ein Behandlungssystem beschrieben, bei der ein Weicheisen-Massekörper durch ein von einer Spule erzeugtes Magnetfeld beschleunigt wird. Der Massekörper überträgt seine kinetische Energie beim Aufprall auf den Stoßweiterleiter auf diesen.

Aus den vorgenannten OE-Patentschriften sind schließlich Behandlungssysteme bekannt, bei denen zur Anregung des Stoßweiterleiters Lichtenergie bzw. die Energie einer Funkenstrecke dient, die über eine Membran auf den Stoßweiterleiter übertragen wird.

Sämtlichen bekannten intrakorporalen Behandlungssystemen, bei denen mit einem Projektil bzw. einem Massekörper gearbeitet wird, ist gemeinsam, daß die lineare Beschleunigungsstrecke für das Projektil und der Stoßweiterleiter streng axial ausgerichtet sind.

Auch bei den aus den OE-Patentschriften bekannten Behandlungssystemen wird ein axial ausgerichteter Aufbau verwendet.

Aufgrund der axialen Ausrichtung des Aufbaus haben die bekannten Behandlungssysteme und insbesondere das bekannte gattungsgemäße intrakorporale Behandlungssystem - wie erfindungsgemäß erkannt worden ist - eine Reihe von Nachteilen:
1. Die Größe der Auslenkung, d.h. die Elongation des Stoßweiterleiters ist über den beaufschlagenden Druck nur bedingt einstellbar.
2. Die Verwendung eines pneumatischen Systems für die Bewegung des Projektils hat zwar den Vorteil, daß das Projektil mit vergleichsweise hoher Frequenz auf das Target auftreffen kann. In einer Reihe von Fällen - beispielweise dann, wenn das zu zerstörende Konkrement nicht in einem Käfig gehalten ist - ist diese hohe "Schußfrequenz" überhaupt nicht erforderlich, da durch die Wucht des auf das Konkrement auftreffenden Stoßweiterleiters das Konkrement verlagert wird, so daß das Behandlungssystem erst neu auf das Konkrement ausgerichtet werden muß.
   In diesen Fällen ist das bekannte intrakorporale Behandlungssystem unnötig aufwendig.
3. Das bekannte intrakorporale Behandlungssystem erlaubt nicht die Integration einer Saug- bzw. Spülleitung.

### Darstellung der Erfindung

Der Erfindung liegt die Aufgabe zugrunde, ein intrakorporales Behandlungssystem zur Zertrümmerung von Konkrementen, wie Steinen, im harnableitenden Trakt, Kalkablagerungen in Gefäßen oder Knochenzement anzugeben, bei dem wenigstens einer der vorgenannten Nachteile des bekannten gattungsgemäßen intrakorporalen Behandlungssystems beseitigt ist, also beispielsweise die Elongation des StoßWeiterleiters einstellbar ist.

Die erfindungsgemäße Lösung dieser Aufgabe ist im Patentanspruch 1 angegeben. Weiterbildungen der Erfindung sind Gegenstand der Ansprüche 2 ff..

Bei einer erfindungsgemäßen Ausgestaltung sind zur Einstellung der Elongation die Längsachsen der Beschleunigungsstrecke und des Stoßweiterleiters gegeneinander versetzt, d.h. sie weisen in Richtung quer zu ihren in der Regel parallel ausgerichteten Längsachsen einen Abstand auf. Hierdurch ist es möglich, zwischen der Beschleunigungsstrecke und dem Stoßweiterleiter ein axial wirkendes Transformationselement anzubringen, das insbesondere ein Hebel, aber auch ein anderer Übertrager sein kann, und das die Bewegungsenergie des beispielsweise als Projektil ausgebildeten Stoßelements auf das proximale Ende des Stoßweiterleiters überträgt. Dieses Transformamationselement dient auch als Target für das Stoßelement.

Durch den Verzicht auf die beim Stand der Technik vorgesehene kollineare Anordnung der Beschleunigungsstrecke für das Stoßelement und des Stoßweiterleiters, durch die erst die Verwendung eines Transformationselementes möglich wird, ist es möglich, bei einer bestimmten - beispielsweise durch den Anwendungsfall vorgegebenen - Bewegungsenergie des Stoßelements die Elongation, d.h. die Auslenkung des Stoßweiterleiters aus seiner Ruhelage, in der er beispielsweise einen Körperstein gerade nicht berührt, auf einen gewünschten Wert einzustellen.

Insbesondere dann, wenn als Transformationselement ein Hebelsystem verwendet wird, ist es möglich, die Hebellänge einstellbar zu machen, so daß die Elongation des Stoßweiterleiters nicht nur herstellerseitig vorgebbar, sondern auch beim Betrieb des erfindungsgemäßen Behandlungssystems eingestellt werden kann.

Darüber hinaus ermöglicht es die erfindungsgemäße Anordnung, bei der die Achsen der Beschleunigungsstrecke und des Stoßweiterleiters gegeneinander versetzt sind, mehrere Beschleunigungsstrecken mit jeweils einem Stoßelement vorzusehen. Dabei können sämtliche Stoßelemente auf ein einziges Transformationselement auftreffen.

Die einzelnen Stoßelemente können dabei so angesteuert werden, daß sie gleichzeitig ihre kinetische Energie auf den Stoßweiterleiter übertragen. Damit kann eine wesentliche Erhöhung der in den Stoßweiterleiter eingekoppelten Energie erreicht werden.

Ferner ist es möglich, daß die kinetische Energie mehrerer Stoßelemente verglichen mit der Pulsdauer der Stöße zeitlich kurz nacheinander in den Stoßweiterleiter eingekoppelt wird. Der Abstand zwischen den einzelnen Stößen kann dabei so bemessen werden, daß sich die einzelnen Stöße überlagern, so daß die zu behandelnde Stelle mit hoher Stoßenergie in schnellem Takt in Art einer "Rütteleinrichtung" beaufschlagt wird.

Beispielsweise ist es möglich als Stoßelemente Projektile einzuetzen, die in einer Beschleunigungsstrecke bewegt werden.

Beispielsweise ist es ähnlich wie beim Stand der Technik möglich, pneumatische Antriebssysteme zu verwenden, bei denen das Projektil mit Druckluft beispielsweise aus einem Druckspeicher von der proximalen Seite her beaufschlagt wird.

Selbstverständlich können zum Antrieb der Projektile auch andere Systeme, wie beispielsweise elektromagnetische Beschleunigungssysteme, piezoelektrische System oder Ultraschallsysteme eingesetzt werden, wie sie u.a. in den einleitend angegebenen Druckschriften beschrieben sind.

In den Ansprüchen 7 und 8 sind verschiedene Möglichkeiten für die Ausbildung der Projektile angegeben. Zur Verminderung der Reibung können das oder die Projektile eine zylindrische Grundform mit einer abgerundeten Stirnfläche und seitlichen Einschnürungen haben oder als Kugeln ausgebildet sein.

Bei einer weiteren erfindungsgemäßen Ausgestaltung ist der Stoßweiterleiter eine Hohlsonde. Der Kanal in der Hohlsonde kann als Saug- oder Spülkanal dienen. Alternativ kann in den Kanal die Sonde eines weiteren Lithotripsiesystems, wie eines elektrohydraulischen Systems eingesetzt sein.
Ferner ist es möglich, am proximalen Ende der Hohlsonde ein akustische Sensor vorzusehen, der eine Stein-/Weichgewebe-Erkennung ermöglicht.

### Kurze Beschreibung der Zeichnung

Die Erfindung wird nachstehend anhand von Ausführungsbeispielen unter Bezugnahme auf die Zeichnung näher bechrieben, in der zeigen:
- Fig. 1: ein erstes Ausführungsbeispiel der Erfindung mit einer Beschleunigungsstrecke,
- Fig. 2: ein zweites Ausführungsbeispiel der Erfindung mit zwei Beschleunigungsstrecken,
und
- Fig. 3: ein drittes Ausführungsbeispiel, bei dem der Stoßweiterleiter eine Hohlsonde ist.

### Darstellung anhand von Ausführungsbeispielen

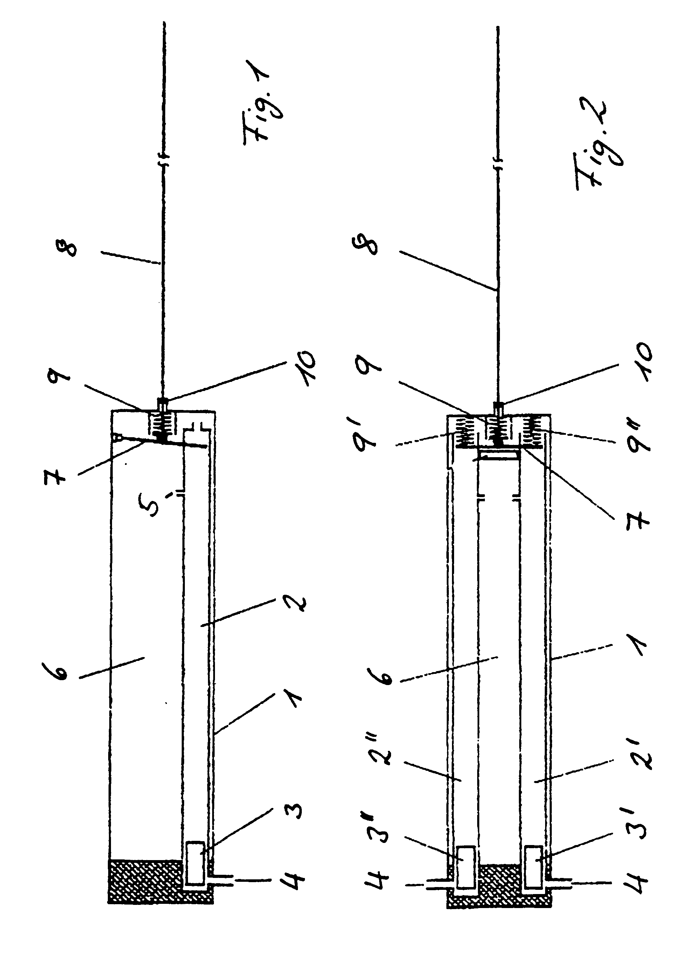

Fig. 1 zeigt ein erstes Ausführungsbeispiel eines erfindungsgemäßen intrakorporales Behandlungssystem. In einem Gehäuse 1 ist eine Stoßeinheit angeordnet, die eine Beschleunigungsstrecke 2 für ein Stoßelement 3 aufweist.

Die Beschleunigungsstrecke 2 besteht aus einem Rohr, das mit einem Einlaß 4 an seinem proximalen Ende und mit einem Auslaß 5 am seinem distalen Ende versehen ist. Das Stoßelement 3 besitzt eine zylindrische Grundform mit abgerundeter Stirnfläche und seitlichen Einschnürungen.

Der Auslaß 5 führt in den Innenraum 6 des Gehäuses 1. Der Einlaß 4 ist in ähnlicher Weise wie in der US-A-5 160 336 beschrieben mit einer Druckluftquelle, die beispielsweise einen Druckluftspeicher aufweisen kann, sowie gegebenenfalls einer Saugpumpe verbunden. Durch den Unterdruck wird die "Rückholung" des Stoßelements 3 aus der distalen Endposition unterstützt. Selbstverständlich ist es aber auch möglich, anstelle eines Druckluftspeichers eine andere Druckluftquelle, einen elektromagnetischen, einen piezoelektrischen, einen magnetorestriktiven Antrieb oder einen Linearantrieb einzusetzen.

Bei dem gezeigten Ausführungsbeispiel wird durch eine geeignete Beaufschlagung des Einlasses 4 mit Über- bzw. Unterdruck wird das Stoßelement 3 derart beschleunigt, daß es auf ein Transformationselement 7 auftrifft, das am Gehäuse 1 angelenkt ist, und das zusätzlich einen als Sonde ausgebildeten Stoßweiterleiter 8 beaufschlagt. Der Stoßweiterleiter 8 ist in den Körper einsetzbar, und wird zur Übertragung des Stoßes an den Behandlungsort durch das Target verschoben. Mit dem Bezugszeichen 9 ist eine Rückhohlfeder bezeichnet. 10 bezeichnet eine Abdichtung am Gehäuse 1. Insbesondere mit "aktiver" Rückholung des Stoßelements 3 durch Unterdruck sind Stoßfrequenzen zwischen 10 und mehr als 30 Hz erreichbar.

Fig. 1 ist zu entnehmen, daß die Längsachsen der Beschleunigungsstrecke 2 und des Stoßweiterleiters 8 gegeneinander versetzt sind. Die Übertragung der kinetischen Energie vom Stoßelement 3 auf den Stoßweiterleiter 8 erfolgt durch das Transformationselement (hier ein Hebel) 7, das als axial wirkendes Transformationselement dient. Durch die kinetische Energie des Stoßelements 3 wird der Stoßweiterleiter 8 um eine Strecke "stoßartig" ausgelenkt, die durch die Geometrie des Transformationselementes 7 und den Anschlag dieses Elementes in dem Rohr 2 bestimmt ist. Durch eine Variation der Hebelverhältnisse kann die Elongation entsprechend dem jeweiligen Anwendungsfall variiert werden.

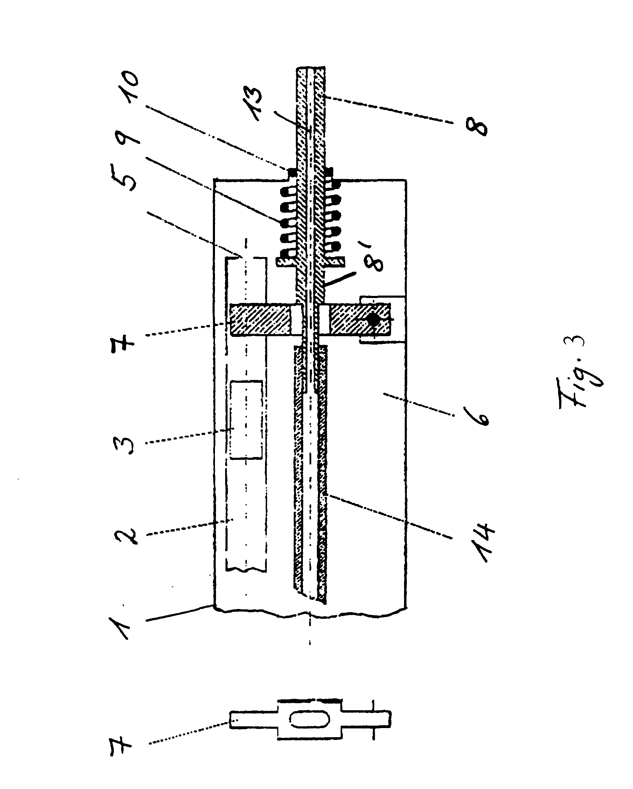

Die Fig. 2 und 3 zeigen weitere Ausführungsbeispiele der Erfindung, bei denen gleiche bzw. entsprechende Teile mit den selben Bezugszeichen wie in Fig. 1 versehen sind, so daß auf eine erneute Vorstellung dieser Teile verzichtet wird.

Das in Fig. 2 gezeigte Ausführungsbeispiel weist zwei Beschleunigungsstrecken 2' und 2" mit jeweils einem Stoßelement 3' bzw. 3" auf. Beide Stoßelemente 3' bzw. 3" treffen auf ein Hebelsystem 7' auf, das die kinetische Energie der Stoßelemente 3' bzw. 3" in den einzigen Stoßweiterleiter 8 einkoppelt.

Die Ansteuerung der Stoßelemente 3', 3" kann dabei derart erfolgen, daß die kinetische Energie der beiden Stoßelemente 3 , 3" gleichzeitig oder kurzzeitig verglichen mit der Pulsbreite eines Stoßes hintereinander in den Stoßweiterleiter 8 eingekoppelt wird.

Bei den in den Fig. 1 und 2 gezeigten Ausführungsbeispielen treibt ein pneumatisches System das bzw. die Stoßelemente 3 an. Selbstverständlich sind auch noch andere Antriebssysteme, wie elektromagnetische oder Piezo-Systeme verwendbar.

Die Fig. 3 zeigt ein drittes Ausführungsbeispiel, bei dem der Stoßweiterleiter 8 eine Hohlsonde ist.

Bei dem in Fig. 3 gezeigten Ausführungsbeispiel sind die Achsen der Beschleunigungsstrecke 2 und des Stoßweiterleiters 8 gegeneinander versetzt. Wie bei dem in Fig. 1 gezeigten Ausführungsbeispiel überträgt ein Transformationselement 7, das als axial wirkendes Transformationselement dient, die Bewegungsenergie des als Projektil ausgebildeten Stoßelements 3 auf das proximale Ende 8' des Stoßweiterleiters 8.
Der in dem Stoßweiterleiter 8 vorgesehene Kanal 13 ist über ein weiterführendes Rohr 14 (z.B. einen Schlauch) beispielsweise mit einer Saug- oder Spülquelle verbunden.

## Patentansprüche

1. Intrakorporales Behandlungssystem zur Zertrümmerung von Konkrementen, wie Steinen im harnableitenden Trakt, Kalkablagerungen in Gefäßen oder Knochenzement, mit
- einer Stoßeinheit, die ein Projektil (3) und eine Beschleunigungsstrecke (2) umfasst, in der das Projektil (3) beschleunigt wird,
- einem Target, welches so angeordnet ist, dass das in der Beschleunigungsstrecke (2) beschleunigte Projektil auf das Target trifft, und
- einem als Sonde ausgebildeten Stoßweiterleiter (8), der in einen menschlichen Körper einsetzbar ist,
- wobei das Target ein Transformationselement ist, das die Bewegungsenergie des Projektils (3) auf das proximale Ende des Stoßweiterleiters (8) axial wirkend überträgt und den Stoßweiterleiter (8) verschiebt, und dadurch einen Stoß auf den Stoßweiterleiter (8) ausübt, und
- der Stoßweiterleiter (8) diesen Stoß an den Behandlungsort überträgt,
**dadurch gekennzeichnet, daß** die Achsen der Beschleunigungsstrecke (2) und des Stoßweiterleiters (8) gegeneinander versetzt sind, und daß das Transformationselement ein Hebel (7) ist.

2. Behandlungssystem nach Anspruch 1,
**dadurch gekennzeichnet, daß** wenigstens eine weitere Beschleunigungsstrecke mit jeweils einem weiteren Projektil (3) vorhanden ist.

3. Behandlungssystem nach Anspruch 2,
**dadurch gekennzeichnet, daß** ein einziger Hebel (7) vorhanden ist.

4. Behandlungssystem nach Anspruch 3,
**dadurch gekennzeichnet, daß** die Hebellänge einstellbar ist, so daß die Elongation des Stoßweiterleiters (8) eingestellt werden kann

5. Behandlungssystem nach einem der Ansprüche 2 oder 3,
**dadurch gekennzeichnet, daß** die kinetische Energie der Projektile (3) gleichzeitig in den Stoßweiterleiter (8) eingekoppelt wird.

6. Behandlungssystem nach einem der Ansprüche 2 oder 3,
**dadurch gekennzeichnet, daß** die kinetische Energie der Projektile (3) kurzzeitig verglichen mit der Pulsbreite eines Stoßes hintereinander in den Stoßweiterleiter (8) eingekoppelt wird.

7. Behandlungssystem nach einem der Ansprüche 1 bis 6,
**dadurch gekennzeichnet, daß** das oder die Projektile (3) eine zylindrische Grundform mit abgerundeter Stirnfläche und seitlichen Einschnürungen haben.

8. Behandlungssystem nach einem der Ansprüche 1 bis 7,
**dadurch gekennzeichnet, daß** das oder die Projektile (3) Kugeln sind.

9. Behandlungssystem nach einem der Ansprüche 1 bis 8,
**dadurch gekennzeichnet, daß** ein pneumatisches System das bzw. die Projektile (3) antreibt.

10. Behandlungssytem nach einem der Ansprüche 1 bis 8,
**dadurch gekennzeichnet, daß** ein elektromagnetisches System das oder die Stoßelemente (3) antreibt.

11. Behandlungssystem nach einem der Ansprüche 1 bis 10,
**dadurch gekennzeichnet, daß** der Stoßweiterleiter (8) eine Hohlsonde ist.

12. Behandlungssystem nach Anspruch 11,
**dadurch gekennzeichnet, daß** der als Hohlsonde ausgebildete Stoßweiterleiter (8) an seinem proximalen Ende in ein weiterführendes Rohr (14) mündet, auf dem das Projektil (3), das mit einer axialen Bohrung versehen ist, gleitet.

13. Behandlungssystem nach Anspruch 11 oder 12,
**dadurch gekennzeichnet, daß** der Kanal (13) in der als Hohlsonde ausgebildetem Stoßweiterleiter (8) als Saug- oder Spülkanal ausgebildet ist.

14. Behandlungssystem nach Anspruch 11 oder 12,
**dadurch gekennzeichnet, daß** in den Kanal (13) die Sonde eines weiteren Lithotripsiesystems, wie eines elektrohydraulischen Systems eingesetzt ist.

15. Behandlungssystem nach Anspruch 11 oder 12,
**dadurch gekennzeichnet, daß** am proximalen Ende des als Hohlsonde ausgebildeten Stoßweiterleiters (8) ein akustischer Sensor vorgesehen ist, der eine Stein-/Weichgewebe-Erkennung ermöglicht.

## Claims

1. System for intracorporeal treatment for crushing concrements such as calculi in the urinary tract, calcareous deposits in vessels or bone cement, comprising
- an impact unit including a projectile (3) and an acceleration path (2) within which said projectile (3) is accelerated,
- a target disposed in such a way that the projectile accelerated in said acceleration path (2) hits on said target, and
- an impact transmitter (8) designed as a probe, which is adapted for being introduced into a human body,
- wherein said target is a transformation element transferring the kinetic energy of said projectile (3) onto the proximal end of said impact transmitter (8) with axial action and displaces said impact transmitter (8), thus producing an impact on said impact transmitter (8), and
- wherein said impact transmitter (8) transfers this impact to the site to be treated,
**characterised in that** the axes of said acceleration path (2) and said impact transmitter (8) are offset relative to each other, and that said transformation element is a lever (7).

2. Treatment system according to Claim 1,
**characterised in that** at least one further acceleration path with a respective further projectile (3) is provided.

3. Treatment system according to Claim 2,
**characterised in that** a single lever (7) is provided.

4. Treatment system according to Claim 3,
**characterised in that** the lever length is adjustable such that the elongation of said impact transmitter (8) may be adjusted.

5. Treatment system according to any of the Claims 2 or 3,
**characterised in that** the kinetic energy of said projectiles (3) is coupled into said impact transmitter (8) simultaneously.

6. Treatment system according to any of the Claims 2 or 3,
**characterised in that** the kinetic energy of said projectiles (3) is coupled into said impact transmitter (8) in succession at short intervals, compared against the pulse width of an impact.

7. Treatment system according to any of the Claims 1 to 6,
**characterised in that** said projectile or projectiles (3) present(s) a cylindrical basic shape with a rounded-off face and lateral necking sites.

8. Treatment system according to any of the Claims 1 to 7,
**characterised in that** said projectile or projectiles (3) is/are (a) sphere(s).

9. Treatment system according to any of the Claims 1 to 8,
**characterised in that** a pneumatic system drives said projectile or projectiles (3).

10. Treatment system according to any of the Claims 1 to 8,
**characterised in that** an electromagnetic system drives said impact body or bodies (3).

11. Treatment system according to any of the Claims 1 to 10,
**characterised in that** said impact transmitter (8) is a hollow probe.

12. Treatment system according to Claim 11,
**characterised in that** said impact transmitter (8) configured as hollow probe opens into a conveying tube (14) at its proximal end, on which said projectile (3) slides, which is provided with an axial bore.

13. Treatment system according to Claim 11 or 12,
**characterised in that** said duct (13) is configured as aspiration or irrigation duct in said impact transmitter (8) designed as hollow probe.

14. Treatment system according to Claim 11 or 12,
**characterised in that** the probe of a further lithotripter system, such as an electro-hydraulic system, is inserted into said duct (13).

15. Treatment system according to Claim 11 or 12,
**characterised in that** an acoustic sensor is provided on the proximal end of said impact transmitter (8) configured as hollow probe, which sensor permits a calculus/soft tissue detection.

## Revendications

1. Système de traitement intracorporeal à broyer des concrétions comme des calculs dans les voies urinaires, des concrétions calcaires dépôts calcaires dans des vaisseaux ou du ciment osseux, comprenant
- une unité de coup renfermant un projectile (3) et un trajet d'accélération (2), au-dedans duquel ledit projectile (3) subit une accélération,
- une cible disposée de façon, que le projectile accéléré dans ledit trajet d'accélération (2) heurte ladite cible, et
- un transmetteur des impulsions (8), conçu sous forme d'une sonde, qui este approprié à être introduit dans un corps humain,
- dans lequel ladite cible constitue un élément transformateur à transférer l'énergie cinétique dudit projectile (3) sur l'extrémité proximale dudit transmetteur des impulsions (8) à effet axial, en déplaçant ledit transmetteur des impulsions (8) et ainsi engendrant un coup sur ledit transmetteur des impulsions (8), et
- dans lequel ledit transmetteur des impulsions (8) transfère ce coup au lieu du traitement,
**caractérisé en ce que** les axes dudit trajet d'accélération (2) et dudit transmetteur des impulsions (8) sont déportés l'un de l'autre, et **en ce que** ledit élément transformateur est un levier (7).

2. Système de traitement selon la revendication 1,
**caractérisé en ce qu'**au moins un autre trajet d'accélération à un autre projectile respectif (3) est disposé.

3. Système de traitement selon la revendication 2,
**caractérisé en ce qu'**un seul levier (7) est prévu.

4. Système de traitement selon la revendication 3,
**caractérisé en ce que** la longueur dudit levier est ajustable de façon que l'élongation dudit transmetteur des impulsions (8) soit ajustable.

5. Système de traitement selon une quelconque des revendications 2 ou 3,
**caractérisé en ce que** l'énergie cinétique desdits projectiles (3) est introduite dans ledit transmetteur des impulsions (8) en même temps.

6. Système de traitement selon une quelconque des revendications 2 ou 3,
**caractérisé en ce que** l'énergie cinétique desdits projectiles (3) est introduit dans ledit transmetteur des impulsions (8) en suite aux intervalles courts, comparé avec la largeur d'impulsion d'un coup.

7. Système de traitement selon une quelconque des revendications 1 à 6,
**caractérisé en ce que** ledit projectile ou lesdits projectiles (3) présente(nt) une forme cylindrique de base à une face arrondie et aux rétrécissements latéraux.

8. Système de traitement selon une quelconque des revendications 1 à 7,
**caractérisé en ce que** ledit projectile ou lesdits projectiles (3) est/sont une/des sphère(s).

9. Système de traitement selon une quelconque des revendications 1 à 8,
**caractérisé en ce qu'**un système pneumatique entraîne ledit projectile ou lesdits projectiles (3).

10. Système de traitement selon une quelconque des revendications 1 à 8,
**caractérisé en ce qu'**un système électromagnétique entraîne ledit ou lesdits corps de coup (3).

11. Système de traitement selon une quelconque des revendications 1 à 10,
**caractérisé en ce que** ledit transmetteur des impulsions (8) est une sonde creuse.

12. Système de traitement selon la revendication 11,
**caractérisé en ce que** ledit transmetteur des impulsions (8), qui est conçu sous forme d'une sonde creuse, s'ouvre dans un tube de transfert (14) à son extrémité proximale, sur lequel ledit projectile (3) glisse, qui est muni d'un bore axial.

13. Système de traitement selon la revendication 11 ou 12,
**caractérisé en ce que** ledit canal (13) est configuré en tant que canal d'aspiration ou d'irrigation dans ledit transmetteur des impulsions (8) conçu sous forme d'une sonde creuse.

14. Système de traitement selon la revendication 11 ou 12,
**caractérisé en ce que** la sonde d'un autre système lithotriteur, par exemple un système électro-hydraulique, est introduite dans ledit canal (13).

15. Système de traitement selon la revendication 11 ou 12,
**caractérisé en ce qu'**un détecteur acoustique est disposé sur l'extrémité proximale dudit transmetteur des impulsions (8) conçu sous forme d'une sonde creuse, ce détecteur permettant une détection calcul/tissu doux.
